# EUROPÄISCHE PATENTSCHRIFT

(11) **EP 2 854 887 B1**
(45) Veröffentlichungstag und Bekanntmachung des Hinweises auf die Patenterteilung: **18.10.2017**
(21) Anmeldenummer: 13729622.4
(22) Anmeldetag: 03.06.2013
(51) Int. Cl.: A61L 29/08, A61L 29/16

(54) **BESCHICHTUNG VON BALLONKATHETERN**
COATING OF BALLOON CATHETERS
REVÊTEMENT DE CATHÉTER À BALLONNET

(30) Priorität: 01.06.2012 DE 102012010800
(43) Veröffentlichungstag der Anmeldung: 08.04.2015
(73) Patentinhaber: Rübben, Alexander, 98000 Monaco (MC); Aachen Scientific International PTE. LTD., 079903 Singapore (SG)
(72) Erfinder: Rübben Alexander, 98000 Monaco (MC)
(74) Vertreter: Schöneborn, Holger
(86) Internationale Anmeldenummer: PCT/EP2013/061352
(87) Internationale Veröffentlichungsnummer: WO 2013/178820

(56) Entgegenhaltungen:
- DE-A1-102007 003 184
- DE-A1-102008 034 826

## Beschreibung

Die Erfindung betrifft ein Verfahren zur Beschichtung des Ballons eines Ballonkatheters, wobei die Oberfläche des Ballons zumindest teilweise mit einer ersten Lösung eines Wirkstoffs benetzt wird und der mit der ersten Lösung eines Wirkstoffs benetzte Teil der Oberfläche des Ballons mit einer Wasser und/oder mindestens einen Alkohol enthaltenden Flüssigkeit benetzt wird. Darüber hinaus betrifft die Erfindung den Ballon sowie den Ballonkatheter selbst.

Die sogenannten "minimalinvasiven Verfahren" nehmen in der Medizin einen immer größeren Stellenwert ein. Zur Behandlung von Gefäßverengungen wie Arteriosklerose wird häufig die perkutane transluminale Angioplastie (PTA) mittels Ballondilatation eingesetzt. Hierbei wird ein Ballonkatheter, der im distalen Bereich einen durch Fluidzufuhr expandierbaren Ballon aufweist, mit Hilfe eines Führungskatheters zur Stenose (Gefäßverengung) gebracht. Hier erfolgt eine Aufweitung des Ballons, wodurch den Blutfluss hemmende Ablagerungen an oder in die Gefäßwand gedrückt werden, so dass ein ungestörter Blutfluss wieder möglich ist. Um eine erneute Gefäßverengung zu verhindern, wird darüber hinaus zum Teil ein Stent implantiert, der das Gefäß offenhalten soll. Nach Zusammenfalten des Ballons wird der Ballonkatheter zurückgezogen und aus dem Gefäßsystem entfernt, während ein evtl. implantierter Stent im Gefäßsystem verbleibt.

Im Rahmen der Angioplastie kann es, nachdem der Ballonkatheter wieder entfernt wurde, zu einer erneuten Verengung des Gefäßes (Restenose) kommen, die auf Zellproliferation zurückzuführen ist, d. h. Zellen wachsen in das Gefäßlumen hinein und sorgen wiederum für eine Behinderung des Blutflusses.

Um dies zu verhindern, werden mit Medikamenten beschichtete Ballonkatheter eingesetzt, mit denen ein Medikament auf die Gefäßinnenwand an der Stelle der Stenose aufgebracht wird, wobei das Medikament meist proliferationshemmend wirkt und ein erneutes Überwuchern und eine Restenose verhindert werden soll.

Typischerweise wird ein in einem Lösungsmittel gelöster Wirkstoff auf die Oberfläche des Ballons des Ballonkatheters aufgebracht, wobei das Lösungsmittel anschließend verdunstet. Der Wirkstoff befindet sich dann als Schicht auf der Oberfläche und kann während der Ballondilatation appliziert werden. Als problematisch hat sich dabei das Haften des Wirkstoffs auf dem Ballon erwiesen.

Möglichkeiten zur Erzielung einer demgegenüber verbesserten Haftung des Wirkstoffs auf der Oberfläche werden beispielsweise in den Dokumenten US 5,102,402 und US 6,129,705 beschrieben. In dem Dokument US 5,102,402 ist ein mit Medikamenten beschichteter Ballonkatheter beschrieben. Dabei werden in einer ersten Variante mit einem Wirkstoff bzw. Medikament gefüllte Mikrokapseln von Falten in der Ballonoberfläche umschlossen und so mechanisch in der jeweiligen Position gehalten. In einer zweiten Variante sind die Mikrokapseln mit Hilfe eines Haftvermittlers auf die Ballonoberfläche geklebt.

In dem Dokument US 6,129,705 wird ein Ballonkatheter beschrieben, dessen Oberfläche eine Beschichtung aufweist, in die mit einem Wirkstoff gefüllte Mikrokapseln vollständig eingebettet sind. Allerdings handelt es sich bei dem Einfüllen des Wirkstoffs in Mikrokapseln und dem anschließenden Befestigen bzw. Einbetten der Mikrokapseln auf der Ballonoberfläche um vergleichsweise aufwendige und damit kostenintensive Verfahren.

Grundsätzlich ist es wünschenswert, wenn die Oberfläche des Ballons des Ballonkatheters eine homogene und reproduzierbare Medikamentenbeladung aufweist und sich gleichzeitig durch eine gleichmäßige Medikamentenabgabe an das umliegende Gewebe im Körper auszeichnet.

Gemäß einem in der WO 2010/009904 A2 beschriebenen Verfahren soll die Oberfläche des Ballons zunächst mit einer ersten Lösung des Wirkstoffs und anschließend mit einer zweiten Lösung desselben Wirkstoffs behandelt werden. Auf diese Weise wird eine sprödere, kreideartige Oberfläche geschaffen, die bei Andrücken des Ballons an die Innenwand des zu behandelnden Gefäßes für eine verbesserte Abgabe des Wirkstoffs sorgt im Vergleich zu Oberflächenbeschichtungen, die nur durch Behandlung mit einer ersten Lösung erzielt wurden.

Als problematisch hat sich in der Vergangenheit jedoch herausgestellt, dass ein erheblicher Teil des Wirkstoffs beim Einbringen des Ballonkatheters in das Blutgefäß oder innerhalb eines verhältnismäßig kurzen Zeitraums danach vom Blutstrom mitgerissen wird und damit für die beabsichtigte Wirkung nicht mehr zur Verfügung steht. Nach Entfernung des Ballonkatheters nimmt die Wirkstoffabgabe sehr rasch ab.

Es stellt sich daher die Aufgabe, ein Verfahren zur Beschichtung des Ballons eines Ballonkatheters bzw. einen Ballon/Ballonkatheter zur Verfügung zu stellen, mit dem eine verbesserte Langzeitwirkung erzielt werden kann.

Diese Aufgabe wird erfindungsgemäß gelöst durch ein Verfahren zur Beschichtung des Ballons eines Ballonkatheters, wobei die Oberfläche des Ballons zumindest teilweise mit einer ersten Lösung eines Wirkstoffs benetzt wird, der mit der ersten Lösung des Wirkstoffs benetzte Teil der Oberfläche des Ballons mit einer Wasser und/oder mindestens einen Alkohol enthaltenden Flüssigkeit benetzt wird und der mit der ersten Lösung und der Wasser und/oder mindestens einen Alkohol enthaltenden Flüssigkeit benetzte Teil der Oberfläche des Ballons mit einer weiteren Lösung benetzt wird, die ein Dextran enthält.

Beim Einsatz des erfindungsgemäßen Ballonkatheters wird dieser in das Blutgefäßsystem eingeführt und durch Aufblasen an die Innenwand des Gefäßes angepresst. Dabei wird ein Großteil der Beschichtung auf die Innenwand übertragen. Nach Ablassen des Drucks und Entfernung des Ballonkatheters aus dem Gefäßsystem dringt der innerhalb der Beschichtung aufgebrachte Wirkstoff nach und nach in das Gefäßgewebe ein, wobei auch 48 h nach Dilatation des Ballons noch Wirkstoffkonzentrationen im behandelten Gefäßgewebe vorliegen, welche 10 bis 50 %, vorzugsweise mindestens 20 % der Konzentration entsprechen, die 2 h nach der Behandlung vorgelegen haben, wie bei Tierversuchen am Schwein nachgewiesen werden konnte.

Es hat sich überraschend herausgestellt, dass die Dextranbeschichtung ähnlich einem Klebstoff auf der Innenwand des behandelten Gefäßes wirkt, d. h. der Wirkstoff haftet erheblich besser auf der Gefäßwand und wird weniger leicht vom Blutstrom mitgerissen. Entsprechend kann der Wirkstoff über einen langen Zeitraum seine Wirkung entfalten und aus der Dextranbeschichtung nach und nach in das Gewebe des Gefäßes gelangen. Es konnte gezeigt werden, dass selbst nach einigen Wochen noch signifikante Wirkstoffkonzentrationen nachweisbar sind.

Das Dextran stellt eine hydrophile Beschichtung dar, die in einer wässrigen Umgebung wie Blut eine gewisse Quellung bzw. Aufweichung erfährt. Dies führt dazu, dass der Wirkstoff während der Ballondilatation gut auf die Innenwand des Gefäßes übertragen wird. Das erfindungsgemäße Verfahren eignet sich insbesondere zum Aufbringen von lipophilen Beschichtungen auf den Ballon. Es hat sich nämlich herausgestellt, dass gerade das hydrophile Dextran gut geeignet ist, dafür zu sorgen, dass lipophile Wirkstoffe während der Ballondilatation effektiv auf die Innenwände der behandelten Gefäße übertragen werden und eine langanhaltende Wirkstoffkonzentration bewirken. Es wird vermutet, dass bei dem erfindungsgemäßen Verfahren zunächst die Wasser und/oder mindestens einen Alkohol enthaltende Flüssigkeit für eine Versprödung der Wirkstoffbeschichtung sorgt und sich die anschließend aufgebrachten Dextran-Moleküle zwischen den Wirkstoffmolekülen ablagern und so für eine homogene Verteilung des Wirkstoffs in der Dextran-Matrix sorgen. Bei durch das erfindungsgemäße Verfahren hergestellten Ballonen wird der Wirkstoff vorteilhafterweise vom im letzten Schritt aufgebrachten Dextran bedeckt.

Unter Ballon im Sinne der Erfindung wird das durch Zufuhr eines Fluids expandierbare Element eines Ballonkatheters verstanden, unabhängig davon, welche Form das expandierbare Element aufweist oder aus welchem Material es besteht. Ballonkatheter sind grundsätzlich hinlänglich im Stand der Technik bekannt und weisen eine langgestreckte, von proximal nach distal verlaufende Kathetersonde sowie einen im distalen Bereich angeordneten Ballon auf. Es handelt sich um einen Katheter, der hinsichtlich seiner Dimensionen auf die Einführung in ein Körperlumen, insbesondere ein (Blut)gefäßsystem, abgestimmt ist. Dabei können die exakten Dimensionen variieren, je nachdem, ob das Blutgefäß bspw. eine Koronararterie, ein intrakranielles Blutgefäß oder eine Unterschenkelarterie ist. Darüber hinaus verfügt der Ballonkatheter über Mittel zur Zuführung eines Fluids zum Ballon. Hierbei kann es sich um ein Zufuhrlumen handeln, das sich über die Länge des Ballonkatheters erstreckt.

Darüber hinaus kann der erfindungsgemäße Ballonkatheter nicht nur der lokalen Wirkstoffeinbringung, sondern zusätzlich der Platzierung eines Stents (Endoprothese) im Körperlumen dienen. Stents sind röhrenartige Stützstrukturen, die in einem Körperlumen, bspw. einem Blutgefäß implantiert werden, um dieses dauerhaft offen zu halten. Derartige Stents können selbstexpandierend sein oder mit Hilfe eines Ballons expandiert werden. Hierzu wird der Stent auf dem Ballon aufgecrimpt und mit Hilfe des Ballonkatheters in das Körperlumen eingebracht. An der vorgesehenen Stelle wird sodann der Ballon durch Zufuhr eines Fluids expandiert, wodurch sich auch der Stent weitet und im Körperlumen verankert wird. Gleichzeitig wird bei Verwendung des erfindungsgemäßen Ballons der Wirkstoff an die Wandung des Körperlumens abgegeben. Schließlich wird der Ballon wieder zusammengezogen und aus dem Körperlumen entfernt, während der Stent im Körperlumen verbleibt.

Bei der Wasser und/oder mindestens einen Alkohol enthaltenden Flüssigkeit handelt es sich insbesondere um eine einen Alkohol und/oder ein Keton enthaltende wässrige Lösung. Die Konzentration des Alkohols und/oder Ketons in der wässrigen Lösung beträgt typischerweise 10 bis 70 % (v/v), bevorzugt 30 bis 65 % (v/v), weiter bevorzugt 50 bis 60 % (v/v) und ganz besonders bevorzugt ca. 55 % (v/v). Verwendbar sind grundsätzlich mit Wasser mischbare Alkohole und Ketone, wobei auch eine Mischung aus mehreren Alkoholen und/oder Ketonen verwendet werden kann, für die dann die oben genannten, bevorzugten Konzentrationsangaben insgesamt gelten. Bevorzugt ist die Verwendung von Ethanol, Methanol, Aceton und/oder Isopropanol. Am meisten bevorzugt ist Ethanol. Weiterhin kann die wässrige Lösung ein azeotropes Lösungsmittelgemisch umfassen, insbesondere ein Alkohol/Wasser-Gemisch, bevorzugt ein Ethanol/Wasser-Gemisch. Denkbar wäre auch, in der Wasser und/oder mindestens einen Alkohol enthaltenden Flüssigkeit eine zusätzliche Menge Wirkstoff vorzusehen, um die Beladung des Ballons mit Wirkstoff zu erhöhen.

Im erfindungsgemäßen Verfahren wird die Oberfläche des Ballons zumindest teilweise mit einer ersten Lösung eines Wirkstoffs benetzt. Anschließend wird der mit der ersten Lösung eines Wirkstoffs benetzte Teil der Oberfläche des Ballons mit einer Wasser und/oder mindestens einen Alkohol enthaltenden Flüssigkeit benetzt.

Durch das Benetzen der Oberfläche des Ballons mit der ersten Lösung eines Wirkstoffs wird auf der Oberfläche eine lackartige, transparente Wirkstoffschicht erzeugt, die als Basis für eine homogene und reproduzierbare Wirkstoffbeladung dient. Während des Benetzens kann das Ballonmaterial, insbesondere im Fall der Verwendung von Methylenchlorid, Lösungsmittel aufnehmen, das unter Normalbedingungen innerhalb weniger Stunden wieder abgegeben wird.

Die durch Behandlung mit der ersten Lösung erzeugte Beschichtung wird durch die Wasser und/oder mindestens einen Alkohol enthaltende Flüssigkeit angegriffen und die Oberfläche wird poröser beziehungsweise versprödet partiell. Die gesamte Beschichtung wird spröder und optisch weniger transparent, also milchiger. Die so erzeugte Oberfläche hat eine kreideartige, unter Umständen auch nicht-kristalline Konsistenz, die einen höheren Wirkstoffabtrag bei Reibung ermöglicht als im Falle einer Beschichtung nur durch Benetzen der Oberfläche des Ballons mit der ersten Lösung des Wirkstoffs.

Auch das Dextran in der weiteren Lösung liegt bevorzugt in einer alkoholischen Lösung vor. Diese kann neben einem oder mehreren Alkoholen insbesondere auch Wasser enthalten. Eine wässrig-alkoholische Lösung ist insofern von Vorteil, als sie das Dextran gut löst, die bereits aufgebrachte Wirkstoffschicht jedoch nicht wieder abträgt. Darüber hinaus sorgt der organische Anteil in der Lösung für eine rasche Trocknung nach der Benetzung. Die Konzentration des Alkohols bzw. der Alkohole in der weiteren Lösung beträgt typischerweise 10 bis 70 % (v/v), bevorzugt 30 bis 65 % (v/v), weiter bevorzugt 50 bis 60 % (v/v) und besonders bevorzugt ca. 55 % (v/v). Als Alkohol verwendbar sind solche Alkohole, die das Dextran lösen. In der Regel sind derartige Alkohole auch mit Wasser mischbar. Bevorzugt sind Ethanol, Methanol und Isopropanol, besonders bevorzugt ist Ethanol.

Die mittlere Molmasse des Dextrans beträgt zweckmäßigerweise 10.000 bis 100.000.000 Da. Als besonders zweckmäßig hat sich eine mittlere Molmasse zwischen 20.000 und 80.000 Da herausgestellt. Der Dextran-Gehalt der weiteren Lösung beträgt vorzugsweise 1 bis 15 Gew.-%, weiter bevorzugt 2 bis 10 Gew.-% und besonders bevorzugt 3 bis 8 Gew.-%.

Bevorzugt sind natürliche Dextrane. Bei Dextranen handelt es sich um hochmolekulare, verzweigte Polymere, die sich aus Glucoseeinheiten zusammensetzen. Sie werden u. a. von Bakterien der Gattung *Leuconostoc* hergestellt. Verwendung finden sie als Blutplasma-Ersatzmittel oder als Träger in der Chromatographie.

Bei dem Dextran kann es sich insbesondere um ein natürliches Dextran handeln. Besonders bevorzugt ist Dextran 40 mit einer mittleren Molmasse von ca. 40.000 Da.

Grundsätzlich kann sowohl die gesamte Ballonoberfläche, als auch nur ein Teil der Ballonoberfläche, beispielsweise der beim Aufdehnen mit dem Gewebe in Kontakt kommende Bereich der Oberfläche, mit dem erfindungsgemäßen Verfahren beschichtet werden. Insbesondere kann der Ballon einen zylinderförmigen Bereich und mindestens einen konusförmigen Bereich umfassen. In diesem Fall kann beispielsweise nur der zylinderförmige Bereich des Ballons mit einem Wirkstoff erfindungsgemäß beschichtet werden oder der zylinderförmige Bereich des Ballons und ein konusförmiger Bereich.

Die erste Lösung kann hinsichtlich des Wirkstoffs gesättigt sein. Als Lösungsmittel können beispielsweise Methylenchlorid, Chloroform, Alkohol, insbesondere Ethanol, Methanol oder Isopropanol, Aceton, Diethylether, flüssige Kohlenwasserstoffe, wie zum Beispiel Pentan, Hexan, Heptan, Cyclohexan oder Octan, Toluol, Tetrahydrofuran (THF) oder Essigester verwendet werden. Möglich ist auch die Verwendung von Lösungsmittelgemischen. Vorzugsweise handelt es sich um eine Lösung des Wirkstoffs in Methylenchlorid.

Sämtliche Benetzungen der Oberfläche des Ballons mit einer Flüssigkeit (erste Lösung, Wasser und/oder mindestens einen Alkohol enthaltende Flüssigkeit oder ein Dextran enthaltende weitere Lösung) können durch Eintauchen des Ballons in die Flüssigkeit erfolgen. Das Eintauchen dauert dabei in der Regel max. 1 min, typischerweise 10 bis 30 s. Der Ballon sollte sich dabei im zumindest teilweise expandierten Zustand befinden. Der Ballon sollte nach dem Eintauchen mit einer Geschwindigkeit von bis zu 10 mm/s aus der ersten Lösung herausgezogen werden. Noch günstiger ist es, wenn das Herausziehen mit einer Geschwindigkeit von weniger als 5 mm/s, vorzugsweise mit einer Geschwindigkeit zwischen 0,5 mm/s und 2 mm/s erfolgt. Durch das langsame Herausziehen wird ein langsames Trocknen der Oberfläche erreicht.

Alternativ zur Benetzung durch Eintauchen kann die Benetzung auch auf andere Weise erfolgen, bspw. durch Besprühen.

Zudem kann die Oberfläche des Ballons vor dem Benetzen mit der ersten Lösung eines Wirkstoffs gereinigt und/oder mit einer Strukturierung beziehungsweise Profilierung versehen werden. Die Oberfläche des Ballons kann beispielsweise mechanisch, thermisch oder chemisch strukturiert beziehungsweise profiliert werden. Insbesondere kann die Oberfläche durch Aufrauen strukturiert bzw. profiliert werden. Vorteilhafterweise werden durch das Vergrößern der Oberfläche des Ballons auf der Oberfläche Vertiefungen mit einer Tiefe von 5 - 50 µm und einem Durchmesser von 5 - 50 µm erzeugt. Die Profilierung sorgt für eine Verbesserung der Aufnahmefähigkeit des Wirkstoffs sowie des Dextrans.

Weiterhin kann die Oberfläche des Ballons nach dem Benetzen mit der ersten Lösung des Wirkstoffs und vor dem Benetzen mit der Wasser und/oder mindestens einen Alkohol enthaltenden Flüssigkeit mit einer zusätzlichen Lösung desselben Wirkstoffs benetzt werden. Dadurch wird die Wirkstoffbeladung erhöht. Auch die Benetzung mit der zusätzlichen Lösung kann bereits dazu führen, dass die gesamte Beschichtung zumindest partiell versprödet. Zudem kann die gesamte Beschichtung optisch weniger transparent, also milchiger werden. Insgesamt bewirkt das Benetzen mit der zusätzlichen Lösung des Wirkstoffs eine höhere Wirkstoffabgabe bei Reibung im Vergleich zu der durch das Benetzen mit der ersten Lösung entstandenen lackartigen Oberfläche.

Grundsätzlich ist es möglich, eine Benetzung des Ballons mit beliebig vielen zusätzlichen Lösungen durchzuführen, wobei nicht jede Lösung den Wirkstoff enthalten muss. Ggf. kann eine zusätzliche Lösung auch einen anderen Wirkstoff enthalten.

Bei der zusätzlichen Lösung kann es sich zum Beispiel um eine Lösung des Wirkstoffs in Methylenchlorid handeln. Vorteilhafterweise sollte die Konzentration der Lösung geringer sein als die der ersten Lösung, beispielsweise 100 mg/ml. Andere Lösungsmittel wie beispielsweise Chloroform oder Ethanol oder Lösungsmittelgemische können ebenfalls verwendet werden. Die Oberfläche des Ballons kann durch Eintauchen des Ballons in die zusätzliche Lösung benetzt werden, ebenso möglich sind jedoch andere Techniken wie Besprühen.

Die Oberfläche des Ballons kann zudem nach dem Benetzen mit der ersten Lösung, dem Benetzen mit der zusätzlichen Lösung, dem Benetzen mit der Wasser und/oder mindestens einen Alkohol enthaltenden Flüssigkeit und/oder nach dem Benetzen mit der ein Dextran enthaltenden weiteren Lösung getrocknet werden. Beispielsweise kann der Ballon eine Längsachse umfassen und während des Trocknens um seine Längsachse gedreht werden. Zur möglichst gleichmäßigen Trocknung kann die Längsachse des Ballons unmittelbar nach dem Benetzen in eine horizontale Lage gebracht werden. Der Ballon kann dann in einem Luftstrom um seine Längsachse gedreht werden.

Bei dem verwendeten Wirkstoff handelt es sich insbesondere um ein Arzneimittel bzw. Medikament, das proliferationshemmend wirkt und das gefäßverengende Überwuchern der durch den Ballon erweiterten Stelle verhindert. Insbesondere kann der Wirkstoff ausgewählt sein aus: Tretinoin, Orphanrezeptoragonisten, Elafinderivate, Corticosteroide, Steroidhormone, Paclitaxel, Rapamycin, Tacrolimus, hydrophobe Proteine sowie zellproliferationsverändernde Substanzen. Es ist auch möglich, Gemische dieser Wirkstoffe zu verwenden. Darüber hinaus können auch Derivate der genannten Wirkstoffe verwendbar sein, wobei unter Derivaten insbesondere Salze, Ester und Amide verstanden werden. Als Steroidhormone können beispielsweise Methylprednisolon, Dexamethason oder Östradiol verwendet werden. Besonders bevorzugt ist die Verwendung von Paclitaxel bzw. Paclitaxelderivaten.

Die Beschichtung des Ballons mit dem Wirkstoff erfolgt vorzugsweise ohne Einsatz von Lösungsvermittlern. Als solche sind z. B. bekannt: Phosphatidylcholin, polyethoxyliertes Rizinusöl, Cardiolipin, Cholesterol sowie Gemische hieraus.

Der erfindungsgemäße Ballon eines Ballonkatheters umfasst eine Oberfläche, die zumindest teilweise eine Beschichtung mit einem Wirkstoff aufweist. Die Beschichtung ist in dem gesamten beschichteten Bereich homogen und spröde ausgestaltet, wobei sich auf der Außenseite der Beschichtung ein Überzug mit dem aufgebrachten Dextran befindet. Das Dextran bedeckt somit sowohl den Wirkstoff als auch den Ballon. Die Oberfläche kann insbesondere eine kreideartige, ggf. auch nicht-kristalline Struktur besitzen. Zudem kann die Oberfläche des Ballons sowohl vollständig als auch nur teilweise beschichtet sein. Insbesondere kann der Ballon einen zylinderförmigen Bereich und mindestens einen konusförmigen Bereich umfassen. In diesem Fall können beispielsweise nur der zylinderförmige Bereich des Ballons oder der zylinderförmige Bereich und ein konusförmiger Bereich mit einem Wirkstoff erfindungsgemäß beschichtet sein. Der erfindungsgemäße Ballon lässt sich mit Hilfe des erfindungsgemäßen Verfahrens herstellen. Er gewährleistet eine homogene und hohe Medikamentenabgabe an das umliegende Gewebe im Körper, die aufgrund der außen aufgebrachten Beschichtung mit einem Dextran lange anhält.

Der erfindungsgemäße Ballonkatheter umfasst den zuvor beschriebenen erfindungsgemäßen Ballon und weist dieselben Vorteile wie der erfindungsgemäße Ballon auf.

### Beispiel

Ein herkömmlicher Ballon eines Ballonkatheters wird zunächst vorgereinigt. Der Ballon wird in expandiertem Zustand in eine Lösung von Paclitaxel in Methylenchlorid getaucht. Die Konzentration des Paclitaxel beträgt 200 mg/ml, die Eintauchzeit 10 s. Anschließend lässt man den langsam herausgezogenen Ballon für 30 s trocknen.

Im zweiten Schritt wird der Ballon im expandierten Zustand für 30 s in eine wässrige Ethanollösung (55 % (v/v)) getaucht. Anschließend lässt man für 120 s trocknen.

Im dritten Schritt wird der Ballon im expandierten Zustand für 10 s in eine Dextran-Lösung getaucht. Das Dextran ist in wässrigem Ethanol (55 % (v/v)) gelöst.

Sämtliche Schritte werden bei Raumtemperatur durchgeführt.

Das Ergebnis ist in den beigefügten Figuren gezeigt.

Es zeigen:
- Fig. 1:: Eine elektronenmikroskopische Aufnahme der Beschichtung nach Eintauchen in die erste Lösung und
- Fig. 2:: eine elektronenmikroskopische Aufnahme der Beschichtung nach Eintauchen in die Wasser und/oder mindestens einen Alkohol enthaltende Flüssigkeit.

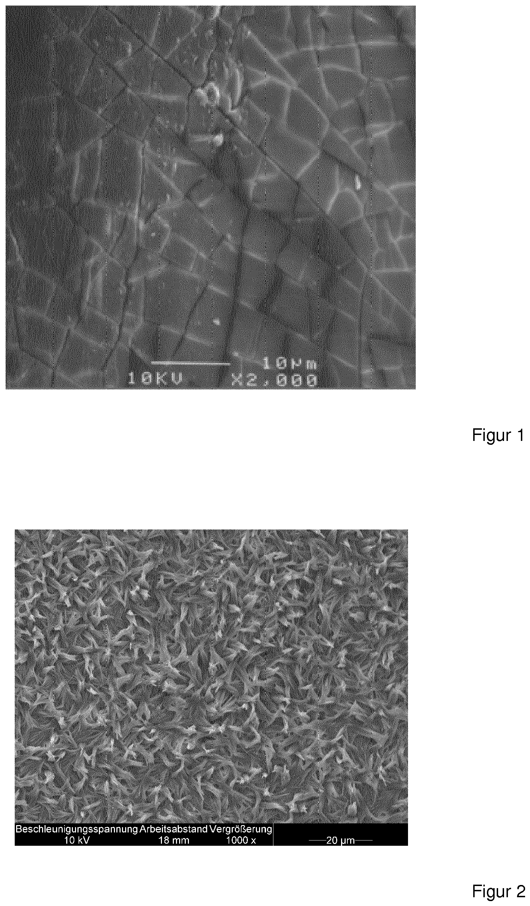

In Figur 1 ist die Oberfläche eines Ballonkatheters nach Eintauchen in die erste, den Wirkstoff enthaltende Lösung dargestellt. Es handelt sich um eine elektronenmikroskopische Aufnahme. Man erkennt eine lackartige Oberfläche.

In Figur 2 ist dieselbe Oberfläche nach Eintauchen in eine wässrige Ethanollösung (55 % (v/v)) dargestellt. Die Oberfläche ist erkennbar spröder, was für eine verbesserte Wirkstoffabgabe an die Gefäßwand sorgt.

## Patentansprüche

1. Verfahren zur Beschichtung des Ballons eines Ballonkatheters, wobei
- die Oberfläche des Ballons zumindest teilweise mit einer ersten Lösung eines Wirkstoffs benetzt wird und
- der mit der ersten Lösung des Wirkstoffs benetzte Teil der Oberfläche des Ballons mit einer Wasser und/oder mindestens einen Alkohol enthaltenden Flüssigkeit benetzt wird,
**dadurch gekennzeichnet, dass** der mit der ersten Lösung und der Wasser und/oder mindestens einen Alkohol enthaltenden Flüssigkeit benetzte Teil der Oberfläche des Ballons mit einer weiteren Lösung benetzt wird, die ein Dextran enthält.

2. Verfahren nach Anspruch 1, **dadurch gekennzeichnet, dass** die Wasser und/oder mindestens einen Alkohol enthaltende Flüssigkeit einen Alkohol und/oder ein Keton enthält.

3. Verfahren nach Anspruch 2, **dadurch gekennzeichnet, dass** die Konzentration des Alkohols und/oder Ketons in der Flüssigkeit 10 bis 70 % (v/v), bevorzugt 30 bis 65 % (v/v), weiter bevorzugt 50 bis 60 % (v/v) beträgt.

4. Verfahren nach Anspruch 2 oder 3, **dadurch gekennzeichnet, dass** die Flüssigkeit Ethanol, Methanol, Aceton und/oder Isopropanol enthält.

5. Verfahren nach einem der Ansprüche 1 bis 4, **dadurch gekennzeichnet, dass** die weitere Lösung einen oder mehrere Alkohole enthält.

6. Verfahren nach Anspruch 5, **dadurch gekennzeichnet, dass** die Konzentration des Alkohols in der weiteren Lösung 10 bis 70 % (v/v), bevorzugt 30 bis 65 % (v/v), weiter bevorzugt 50 bis 60 % (v/v) in Wasser beträgt.

7. Verfahren nach Anspruch 5 oder 6, **dadurch gekennzeichnet, dass** die weitere Lösung Ethanol, Methanol und/oder Isopropanol enthält.

8. Verfahren nach einem der Ansprüche 1 bis 7, **dadurch gekennzeichnet, dass** die mittlere Molmasse des Dextrans 10.000 bis 100.000.000 Da beträgt.

9. Verfahren nach Anspruch 8, **dadurch gekennzeichnet, dass** die mittlere Molmasse des Dextrans 20.000 bis 80.000 Da beträgt.

10. Verfahren nach einem der Ansprüche 1 bis 9, **dadurch gekennzeichnet, dass** der verwendete Wirkstoff ausgewählt ist aus der Gruppe: Tretinoin, Orphanrezeptoragonisten, Elafinderivate, Corticosteroide, Steroidhormone, Paclitaxel, Rapamycin, Tacrolimus, hydrophobe Proteine und/oder zellproliferationsverändernde Substanzen.

11. Verfahren nach einem der Ansprüche 1 bis 10, **dadurch gekennzeichnet, dass** die Oberfläche des Ballons nach dem Benetzen mit der ersten Lösung des Wirkstoffs und vor dem Benetzen mit der Wasser und/oder mindestens einen Alkohol enthaltenden Flüssigkeit mit einer zusätzlichen Lösung desselben oder eines anderen Wirkstoffs benetzt wird.

12. Ballon eines Ballonkatheters, dessen Oberfläche zumindest teilweise eine Beschichtung mit einem Wirkstoff aufweist, erhältlich durch ein Verfahren nach einem der Ansprüche 1 bis 11.

13. Ballonkatheter umfassend einen Ballon nach Anspruch 12.

14. Ballonkatheter nach Anspruch 13, **dadurch gekennzeichnet, dass** der Wirkstoff auf der Oberfläche des Ballons angeordnet ist und das Dextran den Wirkstoff bedeckt.

## Claims

1. Method for coating the balloon of a balloon catheter, wherein
- the surface of the balloon is at least partially wetted with a first solution of an active agent and
- said part of the surface of the balloon wetted with said first solution of an active agent is wetted with a liquid containing water and/or at least one alcohol, **characterized in that** said part of the balloon surface wetted with the first solution and the water and/or at least one alcohol containing liquid is wetted with a further solution that contains a dextran.

2. Method according to claim 1, **characterized in that** the water and/or at least one alcohol containing liquid contains an alcohol and/or a ketone.

3. Method according to claim 2, **characterized in that** the concentration of the alcohol and/or ketone in the liquid ranges between 10 and 70 % (v/v), preferably between 30 and 65 % (v/v), further preferred between 50 and 60 % (v/v).

4. Method according to claim 2 or 3, **characterized in that** the liquid contains ethanol, methanol, acetone and/or isopropanol.

5. Method according to any of claims 1 to 4, **characterized in that** the further solution contains one or several alcohols.

6. Method according to claim 5, **characterized in that** the concentration of the alcohol in water in the further solution ranges between 10 and 70 % (v/v), preferably between 30 and 65 % (v/v), further preferred between 50 and 60 % (v/v).

7. Method according to claim 5 or 6, **characterized in that** the further solution contains ethanol, methanol, and/or isopropanol.

8. Method according to any of claims 1 to 7, **characterized in that** the mean molar mass of the dextran amounts to between 10,000 and 100,000,000 Da.

9. Method according to claim 8, **characterized in that** the mean molar mass of the dextran amounts to between 20,000 and 80,000 Da.

10. Method according to any of claims 1 to 9, **characterized in that** the active agent used is selected from the following group: Tretinoin, orphan receptor agonists, elafin derivatives, corticosteroids, steroid hormones, paclitaxel, rapamycin, tacrolimus, hydrophobic proteins and/or substances modifying cell proliferation.

11. Method according to any of claims 1 to 10, **characterized in that** the surface of the balloon having been wetted with the first solution of the active agent and before being wetted with the water and/or at least one alcohol containing liquid is also wetted with an additional solution containing the same or another active agent.

12. Balloon of a balloon catheter, the surface of which is provided at least partially with a coating comprising an active agent, wherein the balloon is obtainable through a method in accordance with any of claims 1 to 11.

13. Balloon catheter comprising a balloon in accordance with claim 12.

14. Balloon catheter according to claim 13, **characterized in that** the active agent is provided on the surface of the balloon and the dextran covers said active agent.

## Revendications

1. Procédé de revêtement du ballonnet d'un cathéter à ballonnet, selon lequel
- la surface du ballonnet est au moins partiellement mouillée avec une première solution d'un agent actif et
- la partie mouillée avec la première solution de l'agent actif de la surface du ballonnet est mouillée avec un liquide contenant de l'eau et/ou au moins un alcool,
**caractérisé en ce que** la partie mouillée avec la première solution et le liquide contenant de l'eau et/ou au moins un alcool de la surface du ballonnet est mouillée avec une solution supplémentaire qui contient un dextrane.

2. Procédé selon la revendication 1, **caractérisé en ce que** le liquide contenant de l'eau et/ou au moins un alcool contient un alcool et/ou une cétone.

3. Procédé selon la revendication 2, **caractérisé en ce que** la concentration de l'alcool et/ou de la cétone dans le liquide est de 10 à 70 % (v/v), de préférence de 30 à 65 % (v/v), de manière davantage préférée de 50 à 60 % (v/v).

4. Procédé selon la revendication 2 ou 3, **caractérisé en ce que** le liquide contient de l'éthanol, du méthanol, de l'acétone et/ou de l'isopropanol.

5. Procédé selon l'une quelconque des revendications 1 à 4, **caractérisé en ce que** la solution supplémentaire contient un ou plusieurs alcools.

6. Procédé selon la revendication 5, **caractérisé en ce que** la concentration de l'alcool dans la solution supplémentaire est de 10 à 70 % (v/v), de préférence de 30 à 65 % (v/v), de manière davantage préférée de 50 à 60 % (v/v) dans de l'eau.

7. Procédé selon la revendication 5 ou 6, **caractérisé en ce que** la solution supplémentaire contient de l'éthanol, du méthanol et/ou de l'isopropanol.

8. Procédé selon l'une quelconque des revendications 1 à 7, **caractérisé en ce que** la masse molaire moyenne du dextrane est de 10 000 à 100 000 000 Da.

9. Procédé selon la revendication 8, **caractérisé en ce que** la masse molaire moyenne du dextrane est de 20 000 à 80 000 Da.

10. Procédé selon l'une quelconque des revendications 1 à 9, **caractérisé en ce que** l'agent actif utilisé est choisi dans le groupe suivant : la trétinoïne, les agonistes de récepteurs orphelins, les dérivés d'élafine, les corticostéroïdes, les hormones stéroïdiennes, le paclitaxel, la rapamycine, le tacrolimus, les protéines hydrophobes et/ou les substances modifiant la prolifération cellulaire.

11. Procédé selon l'une quelconque des revendications 1 à 10, **caractérisé en ce qu'**après le mouillage avec la première solution de l'agent actif et avant le mouillage avec le liquide contenant de l'eau et/ou au moins un alcool, la surface du ballonnet est mouillée avec une solution supplémentaire du même ou d'un autre agent actif.

12. Ballonnet d'un cathéter à ballonnet, dont la surface comprend au moins en partie un revêtement avec un agent actif, pouvant être obtenu par un procédé selon l'une quelconque des revendications 1 à 11.

13. Cathéter à ballonnet comprenant un ballonnet selon la revendication 12.

14. Cathéter à ballonnet selon la revendication 13, **caractérisé en ce que** l'agent actif est agencé sur la surface du ballonnet et le dextrane recouvre l'agent actif.
